# EUROPÄISCHE PATENTSCHRIFT

(11) **EP 3 587 570 B1**
(45) Veröffentlichungstag und Bekanntmachung des Hinweises auf die Patenterteilung: **31.08.2022**
(21) Anmeldenummer: 18179032.0
(22) Anmeldetag: 21.06.2018
(51) Int. Cl.: C12P 7/18, C12P 7/44, C12P 13/00, C12N 9/80, C12N 9/78, C12P 13/02

(54) **NEUE URETHANASEN FÜR DEN ENZYMATISCHEN ABBAU VON POLYURETHANEN**
NOVEL URETHANASES FOR THE ENZYMATIC DECOMPOSITION OF POLYURETHANES
NOUVELLES URÉTHANASES DESTINÉES À LA DÉGRADATION ENZYMATIQUE DES POLYURÉTHANES

(43) Veröffentlichungstag der Anmeldung: 01.01.2020
(60) Teilanmeldung: 22184632.2
(73) Patentinhaber: Covestro Deutschland AG, 51373 Leverkusen (DE)
(72) Erfinder: Bittner, Natalie, 50226 Frechen (DE); Nefzger, Hartmut, 50259 Pulheim (DE); Behnken, Gesa, 50670 Köln (DE); Jaeger, Gernot, 50733 Köln (DE); Behnken, Swantje, Sacramento, CA 95864 (US); Reisky, Lukas, 50996 Köln (DE)
(74) Vertreter: Levpat

(56) Entgegenhaltungen:
- WO-A1-2006/019095
- DATABASE UniProt [Online] 22. November 2017 (2017-11-22), "Acetyl esterase/lipase aus Planifilum fulgidum", XP002786401, gefunden im EBI accession no. UNIPROT:A0A1I2L9V0 Database accession no. A0A1I2L9V0
- YUKIE AKUTSU-SHIGENO ET AL: "Isolation of a bacterium that degrades urethane compounds and characterization of its urethane hydrolase", APPLIED MICROBIOLOGY AND BIOTECHNOLOGY, SPRINGER, BERLIN, DE, Bd. 70, Nr. 4, 1. April 2006 (2006-04-01), Seiten 422-429, XP019332126, ISSN: 1432-0614, DOI: 10.1007/S00253-005-0071-1
- NAKAJIMA-KAMBE T ET AL: "Microbial degradation of polyurethane, polyester polyurethanes and polyether polyurethanes", APPLIED MICROBIOLOGY AND BIOTECHNOLOGY, SPRINGER, DE, Bd. 51, Nr. 2, 1. Februar 1999 (1999-02-01), Seiten 134-140, XP002379237, ISSN: 0175-7598, DOI: 10.1007/S002530051373

## Beschreibung

Die vorliegende Erfindung betrifft neue Urethanasen für den enzymatischen Abbau von Polyurethanen sowie ein enzymatisches Verfahren für den vollständigen Abbau von Polyurethanen in definierte Monomere.

Polyurethane sind in vielen Bereichen des normalen Lebens etabliert. Sie finden sich zum Beispiel in Weichschäumen (Matratzen, Schwämme, Polstermöbel), Hartschäumen (Isolationsmaterialien, Baustoffe), Thermoplasten (Sportschuhe) oder Beschichtungen (Lacke, Farben, Klebstoffe). Aufgrund der stetig steigenden Nachfrage nach den Produkten werden zunehmend größere Mengen produziert. Parallel dazu wächst das Bedürfnis nach Methoden für möglichst nachhaltiges Recycling von nicht mehr benötigten Polyurethanprodukten, die eine Wiederverwertung von Bausteinen der Polymere erlaubt. Dafür müssen die Bindungen in den Polyurethanen gezielt gespalten werden, um definierte Abbauprodukte erhalten zu können und diese damit wiederverwertbar zu machen.

Neben den physiologischen Funktionen, welche Enzyme in lebenden Organismen erfüllen, können sie auf vielfältige Weise zur Katalyse chemischer Reaktionen außerhalb dieses Kontextes genutzt werden. Hierbei können im Vergleich zu konventionell chemischen Prozessen die Reaktionen bei milderen Bedingungen wie niedrigerer Temperatur, neutralem pH-Wert und ohne die Verwendung aggressiver Chemikalien durchgeführt werden. Dadurch kann Energie gespart, die Bildung von Nebenprodukten minimiert und die Umwelt geschont werden, was zur Reduktion der Betriebskosten beiträgt. Durch den Einsatz von Enzymen wird es teilweise erst ermöglicht, dass labile Edukte umgesetzt werden können (Jaeger, K.-E. & Reetz, M. T. (1998) Microbial lipases form versatile tools for biotechnology. Trends in biotechnology, 16, 396-403). Weiterhin sind Enzyme häufig regio-, stereo- und enantioselektiv, was die Aufreinigung der Produkte deutlich erleichtert und damit die effiziente Synthese schwer zugänglicher Produkte ermöglichen kann (Hasan, F., Shah, A. A. & Hameed, A. (2006) Industrial applications of microbial lipases. Enzyme and Microbial Technology, 39, 235-251).

Das Recycling von Polyurethanen wird hauptsächlich durch thermisches Recycling durchgeführt. Im Allgemeinen läuft dieser Prozess bei sehr hohen Temperaturen und mit sehr langen Reaktionszeiten im Batchprozess, sowie unter Einsatz von Katalysatoren ab. Dabei kann es passieren, dass ein thermischer Abbau der Polymerketten in Crackreaktionen zu unerwünschten und undefinierten Abbauprodukten oder auch die Bildung von Epoxidringen geschieht, welche zu einer hohen Geruchsbelastung und nachteiligen Vernetzungen der Ketten im recyclierten Rohstoff führt und damit insbesondere eine personennahe Wiederverwendung, insbesondere bei der Schaumstoffproduktion für den Möbel- und Matratzenbau unmöglich machen kann. Alternativ wird auch eine vollständige Verbrennung und damit energetische Verwertung durchgeführt, dabei wird zwar Energie gewonnen aber eine effiziente Wiederverwendung der Polymerbausteine entfällt.

Es ist bekannt, dass Polyurethane in gewissem Maße von Bakterien und Pilzen abgebaut werden können. Dabei sind Polyesterpolyurethane deutlich anfälliger gegenüber mikrobiellem bzw. enzymatischem Abbau als Polyetherpolyurethane (Nakajima-Kambe, T., Shigeno-Akutsu, Y., Nomura, N., Onuma, F. & Nakahara, T. (1999) Microbial degradation of polyurethane, polyester polyurethanes and polyether polyurethanes. Applied microbiology and biotechnology, 51, 134-140).

Der Abbau von Polyesterpolyurethanen lässt sich leicht über die Hydrolyse der Esterbindungen realisieren. Der relativ einfache Abbau von Polyestern ist nicht weiter verwunderlich, da Esterbindungen in hydrophoben Substraten in der Natur auch beim Abbau von Lipiden gespalten werden müssen und auch Polyester ohne Urethanbindungen relativ leicht durch Esterasen und Lipasen abgebaut werden können (Marten, E., Müller, R.-J. & Deckwer, W.-D. (2003) Studies on the enzymatic hydrolysis of polyesters I. Low molecular mass model esters and aliphatic polyesters. Polymer degradation and stability, 80, 485-501; Marten, E., Müller, R.-J. & Deckwer, W.-D. (2005) Studies on the enzymatic hydrolysis of polyesters. II. Aliphatic-aromatic copolyesters. Polymer degradation and stability, 88, 371-381.). In verschiedenen Literaturstellen wurden zum Abbau von Polyurethan verwendete Enyzme als Esterasen charakterisiert (Allen, A. B., Hilliard, N. P. & Howard, G. T. (1999) Purification and characterization of a soluble polyurethane degrading enzyme from Comamonas acidovorans. International biodeterioration & biodegradation, 43, 37-41; Blake, R., Norton, W. & Howard, G. (1998) Adherence and growth of a Bacillus species on an insoluble polyester polyurethane. International biodeterioration & biodegradation, 42, 63-73; Crabbe, J. R., Campbell, J. R., Thompson, L., Walz, S. L. & Schultz, W. W. (1994) Biodegradation of a colloidal esterbased polyurethane by soil fungi. International biodeterioration & biodegradation, 33, 103-113; Darby, R. T. & Kaplan, A. M. (1968) Fungal susceptibility of polyurethanes. Applied microbiology, 16, 900-905; Howard, G. T., Norton, W. N. & Burks, T. (2012) Growth of Acinetobacter gerneri P7 on polyurethane and the purification and characterization of a polyurethanase enzyme. Biodegradation, 23, 561-573; Kaplan, A. M., Darby, R. T., Greenberger, M. & Rodgers, M. (1968) Microbial deterioration of polyurethane systems. Dev Ind Microbiol, 82, 362-371; Kay, M., Morton, L. & Prince, E. (1991) Bacterial degradation of polyester polyurethane. International biodeterioration, 27, 205-222; Vega, R. E., Main, T. & Howard, G. T. (1999) Cloning and expression in Escherichia coli of a polyurethane-degrading enzyme from Pseudomonas fluorescens. International biodeterioration & biodegradation, 43, 49-55). Dort gibt es keinen eindeutigen Nachweis auf die Spaltung der Urethanbindung, da die Enzymcharakterisierung in keinem Fall auf Grundlage der Spaltung eines Moleküls mit Urethangruppe durchgeführt wurde.

In vielen Publikationen und Patenten ist der Abbau von Poly(ester)urethanen mit Pilzen oder Bakterien beschrieben. Jedoch zielt der Abbau meist nur auf die relativ leicht zu spaltenden Esterbindungen und wird meist nur durch eine makroskopische Beobachtung des Polymerabbaus belegt. Es gibt hier keinen gesteuerten Abbau von Ester- und Urethanbindungen, wie in der vorliegenden Erfindung, und es ergeben sich häufig lange Abbauzeiten. Diese Veröffentlichungen zeigen, dass Urethanasen weit verbreitete Enzyme sind, jedoch belegen sie nicht deren spezifischen Fähigkeiten, Einsatzmöglichkeiten und Gruppenzugehörigkeit, wie in der vorliegenden Erfindung genutzt. (JP09192633, Tang, Y. W., Labow, R. S., Santerre, J. P. (2003) Enzyme induced biodegradation of polycarbonate-polyurethanes: dose dependence effect of cholesterol esterase. Biomaterials 24 (12), 2003-2011, Vega, R. E., Main, T. & Howard, G. T. (1999) Cloning and expression in Escherichia coli of a polyurethane-degrading enzyme from Pseudomonas fluorescens. International biodeterioration & biodegradation, 43, 49-55)

Zum enzymatischen Abbau von Poly(ester)urethanen ist ein Abbauverfahren bekannt, bei dem im ersten Schritt aus einer Kultivierung von *Comamonas acidovorans* Stämmen eine Esterase gewonnen wird, indem als Kohlenstoffquelle nur Poly(ester)urethan zur Verfügung steht. In einem aufwendigen Aufarbeitungsschritt wird die Esterase abgetrennt und für den Abbau von Poly(ester)urethanen im Batch eingesetzt. Hierbei ergeben sich lange Abbauzeiten in einem mehrstufigen Verfahren und keine Belege für eine spezifische Spaltung der Urethanbindungen. (JP 09201192, JP 10271994)

In verschiedenen Patenten und Publikationen wird der Abbau von Poly(ester)urethanen mit Cutinasen, Esterasen und/oder Lipasen beschrieben. Jedoch zielt der Abbau hier nur auf die relativ einfache Spaltung der Esterbindungen, nicht aber spezifisch auf die Urethanbindungen. Außerdem wird keine gezielte Kombination von Ester- und Urethanbindungen spaltenden Enzymen zur gezielten Steuerung des Abbaus beschrieben. Es ist davon auszugehen, dass die beschriebenen Verfahren nur zu einer geringen oder keiner Spaltung der Urethanbindung führen. Damit können eingesetzte Diamine nicht effizient wiedergewonnen werden. (EP 0968300, US 6,180,381)

In WO 2013/134801 wird der Abbau von aromatischen Polyurethanen, basierend auf Polyetherpolyolen, unter Anwendung eines Enzymes der Klasse EC 3 beschrieben. Dabei sind keine spezifischen Enzymsequenzen angegeben, so dass die Spezifität des Verfahrens in dem Abbau bestimmter Urethanbindungen, sowie der kontrollierten Spaltung von Ester- und gesondert der Spaltung von Urethanbindungen, wie in der vorliegenden Erfindung gezeigt, in dem genannten Patent nicht belegt ist. Weiterhin gibt es keine Beschreibung einer Regelung des pH Wertes der Mischung während des Polymerabbaus, zur Erhaltung der Urethanaseaktivität. Weiterhin ist kein regioselektiver Abbau beschrieben, sowie kein Abbau von aliphatischen Poly(ester)urethanen.

In WO 2006/019095 wird eine Urethanase und durch Protein Engineering gewonnene Varianten dieses Enzyms beschrieben. Das Enzym kann auf TDA oder MDA beruhende Urethanoligomere spalten. Jedoch werden hier weder Bindungen regioselektiv gespalten noch gibt es eine Anwendung in Kombination mit Esterasen zum Abbau von Polymeren. Zudem sind keine weiteren Urethanasen aus der GatA- oder Aes-Familie, oder irgendeiner anderen Gruppe beschrieben.

In der Datenbank "UNIPROT" am EBI ist unter der Nummer A0A1I2L9V0 die Aminosäuresequenz einer putativen Esterase/Lipase offenbart. Die Fähigkeit dieses Enzyms zur Spaltung von Urethanbindungen ist nicht beschrieben.

Der vorliegenden Erfindung lag somit die Aufgabe zugrunde, weitere Enzyme bereitzustellen, die für die enzymatische Spaltung von Urethanbindungen und vorzugsweise für die Verwendung beim vollständigen enzymatischen Abbau von Polyurethanen einsetzbar sind. Weiterhin sollte ein enzymatisches Verfahren bereitgestellt werden, das den Abbau von Polyurethanen in definierte Monomere ermöglicht.

Diese Aufgabe wird durch die in den Patentansprüchen und in der untenstehenden Beschreibung offenbarten Ausführungsformen gelöst.

Die vorliegende Anmeldung offenbart ein Polypeptid mit einer Aminosäuresequenz ausgewählt aus der Gruppe bestehend aus SEQ ID Nr. 3, SEQ ID Nr. 2, SEQ ID Nr. 4, SEQ ID Nr. 5, SEQ ID Nr. 6, SEQ ID Nr. 8, SEQ ID Nr. 10 und Varianten dieser Polypeptide oder ein Polypeptid mit einer Aminosäuresequenz gemäß SEQ ID Nr. 7 oder eine Variante davon, dadurch gekennzeichnet dass das Polypeptid Urethanaseaktivität aufweist.

**Referenz für die erwähnten Polypeptide**

| **SEQ ID Nr.** | **Interne Bezeichnung** | **Bezeichnung in Studie** |
|---|---|---|
| 1 | Enz01 | GatA61 |
| 2 | Enz02 | Aes70 |
| 3 | Enz03 | Aes72 |
| 4 | Enz04 | Aes170 |
| 5 | Enz05 | Aes174 |
| 6 | Enz06 | Aes175 |
| 7 | Enz07 | GatA197 |
| 8 | Enz08 | Aes214 |
| 9 | Enz09 | GatA250 |
| 10 | Enz10 | AesGö56 |
| 11 | Ref01 | SB12 |
| 12 | Ref02 | SB23 |

### Polypeptid

Der Begriff "Polypeptid" ist dem Fachmann gut bekannt. Er bezeichnet eine Kette von wenigstens 50, vorzugsweise wenigstens 70 Aminosäuren, die durch Peptidbindungen miteinander verknüpft sind. Ein Polypeptid kann sowohl natürlich vorkommende als auch synthetische Aminosäuren enthalten. Vorzugsweise enthält es die bekannten proteinogenen Aminosäuren.

Für SEQ ID Nr. 1 bis 5, 9 und 10 wird eine Variante durch Hinzufügung, Deletion oder Austausch von bis zu 10 %, bevorzugt bis zu 5 %, der im jeweiligen Polypeptid enthaltenen Aminosäuren erhalten. Eine bevorzugte Variante von SEQ ID Nr. 7 wird durch Hinzufügung, Deletion oder Austausch von bis zu 5 %, der in SEQ ID Nr. 7 definierten Aminosäuren erhalten. Besonders bevorzugte Varianten der vorgenannten Polypeptide werden durch Hinzufügung, Deletion oder Austausch von bis zu 20, bevorzugt bis zu 10 und noch stärker bevorzugt bis zu 5 Aminosäuren der offenbarten Sequenzen erhalten. Bevorzugte Varianten von SEQ ID Nr. 6 und SEQ ID Nr. 8 werden durch Hinzufügung, Deletion oder Austausch von bis zu 3, stärker bevorzugt bis zu 2 Aminosäuren erhalten. Grundsätzlich können die vorgenannten Modifikationen an jeder beliebigen Stelle des Polypeptids kontinuierlich oder diskontinuierlich erfolgen. Bevorzugt erfolgen sie aber nur am N-Terminus und/oder am C-Terminus des Polypeptids. Jede erfindungsgemäße durch Hinzufügung, Austausch oder Deletion von Aminosäuren erhaltene Variante ist aber durch Urethanaseaktivität wie weiter unten in dieser Anmeldung definiert gekennzeichnet.

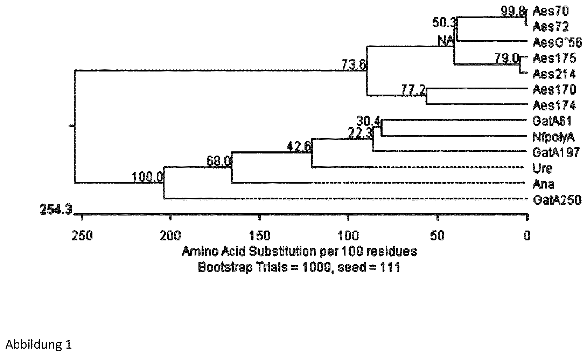

Die Polypeptide, wie sie durch SEQ ID Nr. 3, SEQ ID Nr. 2, SEQ ID Nr. 4, SEQ ID Nr. 5, SEQ ID Nr. 6, SEQ ID Nr. 8 und SEQ ID Nr. 10 definiert sind, bilden eine von dem bisher einzigen bekannten Enzym mit Urethanaseaktivität, Ure, phylogenetisch unterschiedene Gruppe (siehe Abbildung 1). Aus dieser Gruppe waren bisher noch keine Enzyme mit entsprechender Aktivität bekannt. Im Folgenden wird diese Gruppe von Polypeptiden auch als "Aes-ähnlich" bezeichnet.

### Urethanaseaktivität

Der Begriff "Urethanaseaktivität" bezieht sich auf die Fähigkeit eines Polypeptids, die Spaltung einer Urethangruppe enzymatisch zu katalysieren. Pro Mol Urethangruppe entsteht hierbei ein Mol Amin, ein Mol Alkohol und ein Mol CO₂.

Bei der Urethangruppe kann es sich um eine aromatisch oder eine aliphatisch gebundene Urethangruppe handeln. Bei einer aromatisch gebundenen Urethangruppe ist das Stickstoffatom direkt an einen aromatischen Ring gebunden. Bei einer aliphatisch gebundenen Urethangruppe ist das Stickstoffatom an einen Alkylrest gebunden. Vorzugsweise handelt es sich um einen unverzweigten Alkylrest mit wenigstens einem, stärker bevorzugt wenigstens zwei und am stärksten bevorzugt wenigstens drei Kohlenstoffatomen. In einer bevorzugten Ausführungsform der vorliegenden Erfindung ist das Polypeptid mit Urethanaseaktivität in der Lage eine aromatisch gebundene Urethangruppe enzymatisch zu spalten.

Ob ein Polypeptid eine Urethanaseaktivität besitzt, kann durch die Spaltung geeigneter Modellsubstrate überprüft werden.

Für die Fähigkeit zur Spaltung aromatisch gebundener Urethangruppen dient vorzugsweise Ethyl-4-Nitrophenylcarbamat (ENPC) als Modellsubstrat. Die Spaltung wird durch die Bestimmung des Konzentrationsanstiegs von 4-Nitroanilin nachgewiesen. Dies erfolgt vorzugsweise photometrisch bei einer Wellenlänge von 405 nm. Die Enzymaktivität wird vorzugsweise in einem Rektionspuffer mit 100 mM K₂HPO₄/KH₂PO₄, pH 7 mit 6,25 Vol.-% Ethanol in Anwesenheit von 0,2 mg/L ENPC als Substrat bestimmt. Die Inkubation des Enzyms im Reaktionspuffer mit ENPC erfolgt vorzugsweise bei Raumtemperatur und vorzugsweise für 24 Stunden.

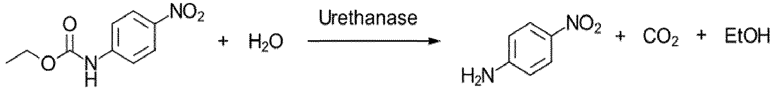

Für die Fähigkeit zur Spaltung aliphatisch gebundener Urethangruppen dient vorzugsweise Ethylphenethylcarbamat (EPEC) als Modellsubstrat. Die Spaltung wird durch die Bestimmung des Konzentrationsanstiegs von Phenethylamin nachgewiesen. Dies erfolgt vorzugsweise durch HPLC. Der verwendete Reaktionspuffer und die Reaktionsbedingungen entsprechen vorzugsweise den oben für ENPC beschriebenen Parametern.

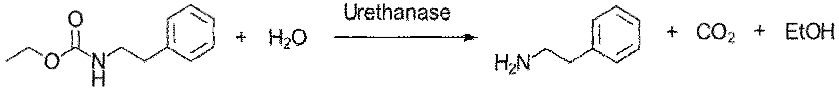

### Enzymatische Spaltung

Der Begriff "enzymatische Spaltung einer Urethangruppe" kennzeichnet, dass die oben beschriebene Spaltung einer Urethangruppe in Anwesenheit eines Polypetids mit Urethanaseaktivität schneller abläuft als bei Inkubation mit dem Reaktionspuffer ohne Enzym unter denselben Reaktionsbedingungen oder bei Inkubation mit dem Reaktionspuffer unter denselben Bedingungen in Anwesenheit eines inaktiven Polypeptids. Als Modell für ein inaktives Polypeptid ist bovines Serumalbumin bevorzugt. Wenn in Anwesenheit eines zu testenden Polypeptids die Spaltung der Urethangruppe schneller abläuft als in einer ansonsten identischen Kontrolle mit BSA, besitzt besagtes Polypeptid Urethanaseaktivität wie in dieser Anmeldung verstanden.

### Verwendung

In einer Ausführungsform betrifft die vorliegende Erfindung die Verwendung eines Polypeptids mit einer Aminosäuresequenz ausgewählt aus der Gruppe bestehend aus SEQ ID Nr. 3, SEQ ID Nr. 2 und Varianten dieser Polypeptide, die durch die Hinzufügung, Deletion oder Austausch von bis zu 10 % der im jeweiligen Polypeptid enthaltenen Aminosäuren erhalten werden und Urethanaseaktivität aufweisen, zur enzymatischen Spaltung von Urethanbindungen.

Alle oben gegebenen Definitionen gelten - soweit nicht explizit anders definiert - auch für diese Ausführungsform.

### Abbau von Urethanen in niedermolekulare Abbauprodukte

In einer weiteren Ausführungsform betrifft die vorliegende Erfindung ein Verfahren zum Abbau von Polyester-Polyurethanen in niedermolekulare Abbauprodukte enthaltend die Schritte
a) Spaltung der im Polyester-Polyurethan enthaltenen Estergruppen; und
b) Behandlung des Polyurethans mit einem Polypeptid, welches Urethanaseaktivität aufweist und eine Aminosäuresequenz ausgewählt aus der Gruppe bestehend aus SEQ ID Nr. 2, SEQ ID Nr. 3 und Aminosäuresequenzen mit wenigstens 90 % Sequenzidentität zu den vorgenannten Sequenzen hat;
mit der Maßgabe, dass die Verfahrensschritte a) und b) in beliebiger Reihenfolge oder auch gleichzeitig durchgeführt werden können.

Vorzugsweise wird der Verfahrensschritt a) vor dem Verfahrensschritt b) durchgeführt.

Verfahrensschritt a) wird vorzugsweise mit einer Lipase durchgeführt. Stärker bevorzugt wird eine Lipase verwendet, die in der Lage ist, Tributyrin zu spalten. Noch stärker bevorzugt wird ein Polypeptid verwendet, das eine Aminosäuresequenz wie in SEQ ID Nr. 11 oder SEQ ID Nr. 12 definiert aufweist oder dessen Aminosäuresequenz wenigstens 90 %, bevorzugt wenigstens 95 %, Sequenzidentität mit einer der beiden vorgenannten Sequenzen aufweist und das in der Lage ist Tributyrin zu spalten. Verfahrensschritt a) wird vorzugsweise unter Reaktionsbedingungen durchgeführt, bei denen die verwendete Lipase Aktivität zeigt. Solche Bedingungen können durch Routineexperimente mit gängigen biochemischen Methoden bestimmt werden.

Da die erfindungsgemäßen Polypeptide mit Urethanaseaktivität ihre maximale Aktivität im neutralen Bereich haben, wird der Verfahrensschritt b) vorzugsweise bei einem pH-Wert zwischen 6,0 und 8,0 durchgeführt. Die Einstellung des pH-Wertes kann durch alle dem Fachmann bekannten und geeigneten Basen erfolgen.

Der Begriff "Polyester-Polyurethan" bezeichnet ein Polyurethan, das aus einem oder mehreren Polyesterpolyolen und einem oder mehreren Isocyanaten aufgebaut ist. Das Polyurethan kann geschäumt oder nicht geschäumt vorliegen. Bevorzugt liegt es geschäumt vor. Um die spezifische Oberfläche zu vergrößern, ist es bevorzugt, das Polyurethan vor Durchführung der Verfahrensschritte a) und b) zu zerkleinern. Dies ist insbesondere bevorzugt, wenn Polyurethan in nicht geschäumter Form eingesetzt werden soll. Das Zerkleinern kann auf alle dem Fachmann geläufigen Arten geschehen, vorzugsweise durch Mahlen, Hobeln, Reißen und Schneiden.

Das Polyurethan enthält als Isocyanatkomponente wenigstens ein aromatisches, aliphatisches oder cycloaliphatisches Isocyanat. Bevorzugt enthält das Polyurethan nur aromatische Isocyanate. Bevorzugte aromatische Isocyanate sind Methylendiphenylisocyanat (MDI), Varianten des MDI mit drei und mehr Kernen und Toluylendiisocyanat. Varianten des MDI mit drei und mehr Kernen sind Nebenprodukte der Synthese und können auch in Polyurethanen enthalten sein. Besonders bevorzugt enthält das abzubauende Polyurethan 2,4-Toluylendiisocyanat und 2,6-Toluylendiisocyanat.

Der Begriff "Polyesterpolyol" ist dem Fachmann bekannt, es handelt sich um Polyester, die im Durchschnitt pro Molekül wenigstens 1,5, bevorzugt wenigstens 1,8 und stärker bevorzugt wenigstens 2,0 Hydroxylgruppen enthalten. Besonders bevorzugt weisen die in dem abzubauenden Polyurethan enthaltenen Polyesterpolyole Funktionalität zwischen 1,5 und 6,0 auf. Sie enthalten als Aufbaukomponenten aromatische und/oder aliphatische Polyole sowie aromatische und/oder aliphatischen Polycarbonsäuren in beliebiger Kombination.

Die niedermolekularen Abbauprodukte der polyesterbasierten Polyurethanschäume weisen vorzugsweise ein Molekulargewicht von höchstens 1.000 g/mol auf. Es handelt sich vorzugsweise um
(i) Amine, die von den bei der Herstellung des betreffenden Polyurethans eingesetzten Isocyanaten abgeleitet sind, Beispielsweise im Falle von 2,4-Toluylendiisocyanat um das 2,4-Toluylendiamin; und
(ii) Alkohole und Carbonsäuren, die zum Aufbau der für die Synthese des betreffenden Polyurethans eingesetzten Polyesterpolyole eingesetzt wurden.

Unter einem "Polyol" wird in dieser Anmeldung jede Verbindung mit wenigstens zwei Hydroxylgruppen verstanden. Besagtes Polyol hat vorzugsweise ein Molekulargewicht von höchstens 300 g/mol. Bevorzugte Polyole, die niedermolekulare Abbauprodukte der polyesterbasierten Polyurethanschäume sind, sind ausgewählt aus der Gruppe bestehend aus Ethylengylcol, Diethylenglykol, Triethylenglycol, Propylenglycol, 1,2- Di-Propylenglycol, Neopentylglykol, Glycerin, 1,1,1-Trimethylolpropan, Saccharose, Sorbitol und Pentaerythrit.

Unter einer "Polycarbonsäure" wird in dieser Anmeldung jede Verbindung verstanden, die wenigstens zwei Carboxylgruppen enthält. Besagte Polycarbonsäure hat vorzugsweise ein Molekulargewicht von höchstens 300 g/mol. Bevorzugte Polycarbonsäuren, die niedermolekulare Abbauprodukte der polyesterbasierten Polyurethanschäume sind, sind ausgewählt aus der Gruppe bestehend aus Bernsteinsäure, Glutarsäure, Adipinsäure, Phthalsäure, Terephthalsäure, Benzoltricarbonsäure, Ölsäure und Ricinolsäure.

Unter einem "Polyamin" wird in dieser Anmeldung jede Verbindung verstanden, die wenigstens zwei Aminogruppen enthält. Besagtes Polyamin hat vorzugsweise ein Molekulargewicht von höchstens 300 g/mol. Bevorzugte Polyamine, die niedermolekulare Abbauprodukte der polyesterbasierten Polyurethanschäume sind, sind ausgewählt aus der Gruppe bestehend aus 4,4'-Methylendiamin, 2,4'-Methylendiamin, 2,2'-Methylendiamin, 2,4- Toluylendiamin, 2,6- Toluylendiamin, Hexamethylendiamin, Isophorondiamin, Xylylendiamin, Pentamethylendiamin, para-Phenylendiamin, Butyldiamin und H12-Methylendiamin. Besonders bevorzugt sind die Polyamine ausgewählt aus der Gruppe bestehend aus 4,4'-Methylendiamin, 2,4'-Methylendiamin, 2,2'-Methylendiamin, 2,4-Toluylendiamin und 2,6-Toluylendiamin.

Das erfindungsgemäße Verfahren ermöglicht ein effektives Recycling von Polyurethanen in zweierlei Hinsicht: (i) Das Verfahren selbst benötigt aufgrund der milden Reaktionsbedingungen keinen hohen Energieeinsatz und ermöglicht (ii) die stoffliche Verwertung des Polyurethans, da definierte Abbauprodukte entstehen, die ihrerseits wertvolle chemische Grundstoffe sind.

Im Vergleich dazu erfolgt die thermische Glykolyse, die zurzeit die gängigste Chemolyse zum Recycling von Polyurethan darstellt und bereits industriell umgesetzt ist, bei sehr hohen Temperaturen. Dabei liegt der Fokus auf der Gewinnung der Polyole, während die Amine als Störfaktor abgetrennt und nicht zurückgewonnen werden. Bei der nicht-enzymatischen Hydrolyse werden sowohl Polyole als auch Amine als Produkte gewonnen. Dieses Verfahren erfolgt jedoch bei hohen Temperaturen und hohen Umgebungsdrücken.

### Übersicht über die Abbildungen:

- Abbildung 1:: Ergebnis der phylogenetischen Analyse der in der vorliegenden Anmeldung offenbarten Aminosäuresequenzen

Die folgenden Ausführungsbeispiele dienen nur dazu, die Erfindung zu erläutern. Sie sollen den Schutzumfang der Patentansprüche in keiner Weise beschränken.

### Beispiele

### Test der Enzymaktivität mit ENPC

0,2 mg/ml ENPC wurden in 100 mM KH₂PO₄/K₂HPO₄ bei pH 7,0 mit 6,25 Vol.-% Ethanol bei Raumtemperatur und 900 rpm auf dem Plattenschüttler "MTS 2/4" (IKA, Staufen) für 24 Stunden inkubiert.

Nach dem Filtrieren der Proben wurden jeweils 100 µL in transparente 96-well-Platten mit flachem Boden "UV-Star" (Greiner Bio-One, Frickenhausen) übertragen und die Extinktion bei 405 und 480 nm bestimmt. Der Wert bei 480 nm wurde gemessen, da 4-Nitroanilin hier nicht mehr signifikant absorbiert und es somit bei hohen Werten für beide Wellenlängen sehr wahrscheinlich war, dass nicht 4-Nitroanilin sondern ein anderer Stoff für die Extinktion bei 405 nm verantwortlich war.

Bei der Hydrolyse durch Urethanasen wird das fast farblose ENPC zu 4-Nitroanilin, CO₂ und Ethanol gespalten, wobei 4-Nitroanilin bei 405 nm im Mikrotiterplatten-Photometer "Infinite M1000PRO" (Tecan, Männedorf, Schweiz) detektiert werden konnte. Zur Steuerung des Photometers wurde die Software "i-control" (Tecan, Männedorf, Schweiz) in der Version 3.4.2.0 verwendet. Zudem wurde 4-Nitroanilin mit der Methode "Dabsylamin" per HPLC detektiert.

### Hochdruckflüssigchromatographie (HPLC)

Die Hochdruckflüssigchromatographie wurde an einem Gerät der 1100er Serie von Agilent Technologies (Santa Clara, USA) mit *Autosampler* und DAD (*diode array detector)* für UV und den sichtbaren Bereich des Lichtes durchgeführt. Für alle Messungen wurde die Säule "Zorbax XDB-C18" mit einer Partikelgröße von 3,5 µm und den Abmessungen 4,6 x 75 mm (Agilent Technologies, Santa Clara, USA) verwendet. Bei allen Methoden wurden 5 µL Probe injiziert und die Säule auf 40 °C temperiert. Der Fluss lag generell bei 1,5 mL/min. Wegen der Verwendung einer Umkehrphasensäule wurde bei allen Methoden mit steigenden Konzentrationen an organischem Lösemittel eluiert.

Zur Detektion und Quantifizierung von dabsylierten aliphatischen Aminen und Urethanen wurde die Methode "Dabsylamin" verwendet. Aromatische Amine und Urethane konnten wegen der hohen Eigenabsorption ohne Derivatisierung mit dieser Methode quantifiziert werden. Als Laufmittel wurde neben AcN 10 mM Natriumphosphatpuffer pH 7,0 verwendet, dem zum Schutz vor mikrobiellem Wachstum 0,005% (w/v) Natriumazid zugegeben wurde. Um Druckproblemen durch verunreinigte Pumpenventile vorzubeugen wurde dem AcN später 5% (v/v) ddH₂O zugegeben und die Methode angepasst ("Dabsylamin95"). Zur Bestimmung der MDEC-Menge aus den enzymkatalysierten Reaktionen von 4,4'-MDA mit EC wurde die Methode "Dabsylamin-12-MeOH" verwendet, wobei die wässrige Komponente angesäuert ist und die dadurch protonierten aromatischen Amine sehr früh eluieren. Zur Untersuchung der Reaktionen von 4,4'-MDA mit DMC, 2,4-TDA mit DMC und 2,4-TDA mit EC wurde die Methode "Dabsylamin95-H2O" genutzt, welche sich von "Dabsylamin95" lediglich durch die Verwendung von reinem ddH₂O anstatt Puffer unterscheidet. Die Analyse der Daten erfolgte mit der Software "OpenLAB CDS ChemStationLC" in der Version A.02.09[017] (Agilent Technologies, Santa Clara, USA).

**Dabsylamin: Laufmittel: AcN und 10 mM Na₂HPO₄/NaH₂PO₄, pH 7,0**

| t [min] | AcN |
|---|---|
| 0 | 5 |
| 6,5 | 85 |
| 8,0 | 5 |
| 10,0 | 5 |

**Dabsylamin95: Laufmittel: AcN mit 5% (v/v) ddH₂O und 10 mM Na₂HPO₄/NaH₂PO₄, pH 7,0**

| t [min] | % AcN (+5% (v/v) ddH₂O) |
|---|---|
| 0 | 5 |
| 6,5 | 90 |
| 8,0 | 5 |
| 10,0 | 5 |

**Dabsylamin-12-MeOH-lang: Laufmittel: Methanol und ddH₂O mit 0,1% (v/v) Methansäure**

| t [min] | % Methanol |
|---|---|
| 0 | 5 |
| 2,5 | 35 |
| 8,0 | 70 |
| 8,5 | 85 |
| 10,0 | 5 |
| 12,0 | 5 |

| **SEQ ID Nr.** | **Bezeichnung in Studie** | **Hydrolyse von ENPC** |
|---|---|---|
| 1 | GatA61 | + |
| 2 | Aes70 | + |
| 3 | Aes72 | + |
| 4 | Aes170 | + |
| 5 | Aes174 | + |
| 6 | Aes175 | + |
| 7 | GatA197 | + |
| 8 | Aes214 | + |
| 9 | GatA250 | + |
| 10 | AesGö56 | + |
| 11 | SB12 | + |
| 12 | SB23 | + |

### Test der Enzymaktivität mit EPEC

Der Versuch wurde wie für ENPC beschrieben durchgeführt. Das entstehende Phenethylamin wurde durch HPLC nachgewiesen wie oben beschrieben.

| **SEQ ID Nr.** | **Bezeichnung in Studie** | **Hydrolyse von EPEC** |
|---|---|---|
| 1 | GatA61 | + |
| 2 | Aes70 | - |
| 3 | Aes72 | + |
| 4 | Aes170 | - |
| 5 | Aes174 | + |
| 6 | Aes175 | - |
| 7 | GatA197 | + |
| 8 | Aes214 | + |
| 9 | GatA250 | + |
| 10 | AesGö56 | - |
| 11 | SB12 | + |
| 12 | SB23 | + |

### Phylogenetische Analyse der Enzyme

Zur Erstellung von Stammbäumen zur Verwandtschaft der Urethanasen wurde das Programm "MegAlign" (DNASTAR, Madison, USA) in der Version 10.1.0 verwendet. Stammbäume wurden mit den Standardeinstellungen unter Nutzung von "ClustalW" erstellt.

Für die Erstellung von *Alignments* verschiedener Proteine wurde das Programm "Clustal Omega" verwendet (Sievers *et al.,* 2011).

Die Datenbanksuche nach Proteinsequenzen wurde mit BLASTP durchgeführt (Altschul *et al.,* 1990).

*Open Reading Frames* (ORFs) in sequenzierten Metagenomsequenzen wurden mit dem Onlineprogramm "ORF-Finder" von NCBI (Wheeler *et al.,* 2007) ausfindig gemacht.

Identische Hydrolasegene wurden auf einen Vertreter reduziert und alle Sequenzen mit ORFs untersucht, um die kompletten Sequenzen der Gene zu erhalten. Bei der Suche wurden auch alternative Startcodons zugelassen. Hierbei fiel auf, dass für das Gen aus pLip214 ein *N*-terminaler Bereich mit Ähnlichkeit zu *aes* vorhanden war, wobei kein Startcodon gefunden werden konnte. Dieser Genabschnitt lag nicht am Rand des Inserts des Metagenomvektors, was ein verkürztes Gen erklären könnte. Für weitere Analysen wurde als Sequenz für dieses Gen der Bereich mit Ähnlichkeit zu *aes* ohne Startcodon genutzt. Die identifizierten putativen Urethanasegene wurden *in silico* translatiert und über BLASTP mit der NCBI-Datenbank abgeglichen. Die putativen Urethanasen wurden gemäß ihrer Nummer in der Lipasebank und der Ähnlichkeit zu GatA oder Aes benannt.

Um die einzelnen Mitglieder der beiden identifizierten Urethanasegruppen (GatA und Aes) untereinander zu vergleichen wurde jeweils ein *Alignment* mit dem Programm "Clustal Omega" gebildet und zusätzlich ein Stammbaum mit der Programm "MegAlign" erstellt, wobei für den Stammbaum ein geneimsames *Alignment* beider Gruppen erstellt wurde. Es wurden auch die Sequenzen für die Enzyme aus der Literatur (Ure, Ana und NfpolyA), die alle Ähnlichkeit mit GatA aufweisen, mit in den Sequenzvergleich einbezogen.

Der Stammbaum ist in Abbildung 1 dargestellt. Es zeigt sich, dass beide Gruppen in unterschiedlichen Ästen lokalisiert sind, wobei die Ähnlichkeitsverhältnisse innerhalb der beiden Gruppen teilweise nicht so deutlich waren, was an geringeren *Bootstrapping-Werten* an den Knotenpunkten zu erkennen ist. Innerhalb der GatA-Gruppe scheinen größere Unterschiede zu existieren als innerhalb der Aes-Gruppe, was an den größeren Astlängen in dieser Gruppe zu erkennen ist. Vor allem Aes70 und Aes72 sowie Aes175 und Aes214 weisen eine sehr hohe Ähnlichkeit auf, was sowohl durch die relativ kurzen Äste im Stammbaum als auch durch das Auffinden des gleichen Proteins mit der größten Ähnlichkeit bei der BLASTP-Suche äußerte.

### Herstellung des Polyurethanschaums für die Abbauversuche

In für die Herstellung von Polyurethanschaumstoffen üblicher Verarbeitungsweise nach dem Einstufenverfahren wurden die unten aufgeführten Einsatzstoffe miteinander zur Reaktion gebracht. Die Rohdichte betrug 38 kg/m3 (DIN EN ISO 845 in der Fassung vom Oktober 2009), die Stauchhärte bei 40% Stauchung 3,5 kPa (DIN EN ISO 3386-1 in der Fassung vom Oktober 2015)

### Rezeptur:

| | |
|---|---|
| 100 Teile | Desmophen 2200B |
| 3 Teile | Wasser |
| 19 Teile | Desmodur T80 |
| 19 Teile | Desmodur T65 |
| 0,7 Teile | N,N'-Dimethylpiperazin |
| 1 Teil | Tegostab 8325 |

### Rohstoffe:

Desmophen^{®}2200B, Fa. Covestro Deutschland AG; verzweigtes Polyesterpolyol auf Basis Adipinsäure, Diethylenglykol und 1,1,1-Trimethylolpropan mit einer Hydroxylzahl von ca. 60 mg KOH/g.

Desmodur^{®}T80, Fa. Covestro Deutschland AG; Isomerengemisch aus 2,4- und 2,6-Toluylendiisocyanat mit einem Mischungsverhältnis von ca. 80:20.

Desmodur^{®}T65, Fa. Covestro Deutschland AG; Isomerengemisch aus 2,4- und 2,6-Toluylendiisocyanat mit einem Mischungsverhältnis von ca. 67: 33.

N,N'-Dimethylpiperazin, Katalysator der Fa. abcr GmbH

Tegostab^{®}B 8325, Schaumstabilisator der Fa. Evonik

Wasser; entionisiertes Wasser

Die Rezeptur kann mit Kennzahlen von 90 bis 115 ausgeführt werden. Mit Kennzahl wird das mit 100 multiplizierte molare Verhältnis von Isocyanatgruppen zu isocyanatreaktiven Gruppen bezeichnet.

### Abbau von Polyurethanschaum

Als Substrat wurde ein mit Toluylendiisocyanat hergestelltes Polyesterpolyurethan verwendet. Der Abbau erfolgte in zwei Reaktionsschritten. Zunächst wurde der Schaum mit einer Lipase inkubiert. Die hierbei entstehenden Oligomere wurden nach Neutralisation mit einer Urethanase in Monomere aufgespalten.

Im ersten Schritt wurden 1 g des Schaumes mit 20 mL Kaliumphosphatpuffer pH 7,0 und ca. 30 mg CalB-Lyophilisat "Chirazyme L2" (Roche, Basel, Schweiz) (hier als SEQ ID Nr. 12 bezeichnet) in ein 50-ml Zentrifugenröhrchen gegeben und bei 37 °C und 200 rpm für 5 Tage inkubiert. Stücke der Schaumreste wurden im Vergleich zu einer Negativkontrolle ohne Enzym mit dem Mikroskop "MH2" (Olympus, Hamburg) fotografiert. Anschließend wurde die trübe Lösung bei 25 °C und 4000 rpm in einer Großraumzentrifuge für 10 Minuten zentrifugiert. Der klare Überstand wurde mit 1 M NaOH auf pH 7,0 eingestellt. Nach ca. 6 Stunden bei Raumtemperatur wurde der leicht gefallene pH-Wert erneut auf 7,0 titriert und die Lösung sterilfiltriert. Die löslichen Oligomere wurden bis zur Verwendung bei 4 °C gelagert.

Zur weiteren Verwendung wurden die löslichen Oligomere in 1,5-mL-Reaktionsgefäße gegeben, mit 20 µL DMF und 150 µL des für die jeweilige Urethanase optimalen Puffers (100 mM Natriumphosphatpuffer, eingestellt auf das jeweilige Optimum der Urethanse im Bereich pH 6,0 bis pH 8,0) versetzt. Anschließend wurden jeweils 30 µL der unverdünnten, aufgereinigten Urethanasen zugegeben und die Ansätze bei 30 °C und 1000 rpm auf dem Thermoblock geschüttelt. Als Negativkontrolle diente ein Ansatz mit Enzymlagerpuffer. Nach drei Tagen wurden die Ansätze über Filterplatten mit PVDF-Membran und 0,2 µm Porengröße (Corning, Kaiserslautern) gefiltert und das Filtrat per HPLC mit der Methode "Dabsylamin95" auf entstandenes 2,4- und 2,6-Toluylendiamin hin untersucht.

Bereits nach der Reaktion im Ansatz mit dem CalB-Lyophilisat war makroskopisch im Vergleich zu einer Negativkontrolle ohne Enzym zu erkennbar, dass der Schaum jegliche Struktur verloren hatte und als trübe Suspension mit kleinen Schaumpartikeln vorlag. Der zu Anfang des Experimentes fast vollständig vom Schaum aufgenommene Puffer beinhaltete später die gesamte Schaummasse als zersetzte Partikel. Bei der HPLC-Analyse zeigten sich deutliche Peaks, die den gebildeten Oligomeren zugeschrieben wurden, aber keine Peaks, die auf die Bildung von Toluylendiamin (TDA) hindeuten würden (Daten nicht gezeigt).

Die Oligomerlösung wurde mit allen exprimierten Urethanasen und SEQ ID Nr. 12 versetzt und anschließend per HPLC auf die Bildung von TDA untersucht. Dieses konnte für die Ansätze mit SEQ ID Nr. 7 und SEQ ID Nr. 3 nachgewiesen werden, wobei die gemessene Menge von 2,6-TDA in beiden Ansätzen etwa gleich war und die Bildung von 2,4-TDA im Ansatz mit Enz03 deutlich stärker ausfiel. Mit SEQ ID Nr. 3 wurden 0,057 g/L 2,4-TDA und 0,025 g/L 2,6-TDA gebildet, während SEQ ID Nr. 7 zur Bildung von 0,0075 g/L 2,4-TDA und 0,024 g/L 2,6-TDA führte. Weiterhin zeigte sich mit diesen beiden Enzymen im Gegensatz zu den anderen Ansätzen eine Veränderung und generelle Verkleinerung der Oligomerpeaks. Im Falle von SEQ ID Nr. 7 konnte bereits TDA ohne vorherige Vorbehandlung aus dem Polyester-PU-Schaum abgespalten werden, während dies im Falle von SEQ ID Nr. 3 erst durch die Bereitstellung neutralisierten von Oligomeren nach vorheriger Esterspaltung möglich war. Die Tatsache, dass die als Oligomerpeaks identifizierten Produktpeaks aus der Hydrolyse mit SEQ ID Nr. 12 nach weiterer Behandlung mit Urethanasen drastisch verkleinert wurden, wobei eine deutliche TDA-Bildung auftrat, bestätigte, dass es sich bei diesen Peaks um Oligomere handelte.

Es konnte also gezeigt werden, dass unlöslicher auf TDI-basierender aus Polyester-Polyurethan Schaum durch eine Kombination von zwei Reaktionsschritten in seine Monomere aufgespalten werden kann. In einem ersten Schritt wurde unter Einsatz der Lipase CalB über die Hydrolyse der Esterbindungen der PU Schaum vorverdaut. Nach Neutralisation wurden die frei gewordenen Oligomere als Substrat für die überexprimierten Urethanasen eingesetzt. Dabei wurden die Urethanbindungen hydrolysiert und TDA konnte als Monomer nachgewiesen werden.

Es zeigt sich also abschließend, dass eine Kombination aus hydrolytischer Spaltung der Esterbindungen mittels Lipasen, Neutralisation der Oligomerlösung sowie anschließende hydrolytische Spaltung der Urethanbindungen einen vollständigen Abbau von Polyurethanen in definierte Monomere erlauben.

## Patentansprüche

1. Verwendung eines Polypeptids mit einer Aminosäuresequenz ausgewählt aus der Gruppe bestehend aus SEQ ID Nr. 3, SEQ ID Nr. 2 und Varianten dieser Polypeptide, die durch die Hinzufügung, Deletion oder Austausch von bis zu 10 % der im jeweiligen Polypeptid enthaltenen Aminosäuren erhalten werden und Urethanaseaktivität aufweisen, zur enzymatischen Spaltung von Urethanbindungen.

2. Verwendung gemäß Anspruch 1, wobei die Urethangruppe aromatisch gebunden ist.

3. Verfahren zum Abbau von Polyester-Polyurethanen in niedermolekulare Abbauprodukte enthaltend die Schritte
a) Spaltung der im Polyester-Polyurethan enthaltenen Estergruppen; und
b) Behandlung des Polyurethans mit einem Polypeptid, welches Urethanaseaktivität aufweist und eine Aminosäuresequenz ausgewählt aus der Gruppe bestehend aus SEQ ID Nr. 2, SEQ ID Nr. 3 und Aminosäuresequenzen mit wenigstens 90 % Sequenzidentität zu den vorgenannten Sequenzen hat;
mit der Maßgabe, dass die Verfahrensschritte a) und b) in beliebiger Reihenfolge oder auch gleichzeitig durchgeführt werden können.

4. Das Verfahren nach Anspruch 3, wobei der Verfahrensschritt a) vor dem Verfahrensschritt b) durchgeführt wird.

5. Das Verfahren nach einem der Ansprüche 3 oder 4, wobei als Verfahrensprodukte Polyole, Polycarbonsäuren und Polyamine entstehen.

6. Das Verfahren nach Anspruch 5, wobei wenigstens ein Polyamin ausgewählt aus der Gruppe bestehend aus 4,4'-Methylendiamin, 2,4'-Methylendiamin, 2,2'-Methylendiamin, 2,4-Toluylendiamin und 2,6- Toluylendiamin entsteht.

7. Das Verfahren nach Anspruch 5, wobei wenigstens ein Polyol ausgewählt aus der Gruppe bestehend aus Ethylengylcol, Diethylenglykol, Triethylenglycol, Propylenglycol, 1,2- Di-Propylenglycol, Neopentylglykol, Glycerin, 1,1,1-Trimethylolpropan, Saccharose, Sorbitol und Pentaerythrit entsteht.

8. Das Verfahren nach Anspruch 5, wobei wenigstens eine Polycarbonsäure ausgewählt aus der Gruppe bestehend aus Bernsteinsäure, Glutarsäure, Adipinsäure, Phthalsäure, Terephthalsäure, Benzoltricarbonsäure, Ölsäure und Ricinolsäure entsteht.

9. Das Verfahren nach einem der Ansprüche 3 bis 8, wobei der Verfahrensschritt a) mit einer Lipase durchgeführt wird.

10. Das Verfahren nach Anspruch 9, wobei die Lipase eine Aminosäuresequenz wie in SEQ ID Nr. 11, SEQ ID Nr. 12 oder einer Variante der vorgenannten Sequenzen mit wenigstens 90 % Sequenzidentität zu SEQ ID Nr. 11 oder SEQ ID Nr. 12 hat.

## Claims

1. Use of a polypeptide having an amino acid sequence selected from the group consisting of SEQ ID No. 3, SEQ ID No. 2 and variants of said polypeptides that are obtained by adding, deleting or exchanging up to 10% of the amino acids present in the respective polypeptide and have urethanase activity in the enzymatic cleavage of urethane linkages.

2. Use according to Claim 1, wherein the urethane group is aromatically attached.

3. Process for breaking down polyester polyurethanes into low-molecular-weight breakdown products, comprising the steps of
a) cleaving the ester groups present in the polyester polyurethane; and
b) treating the polyurethane with a polypeptide that has urethanase activity and has an amino acid sequence selected from the group consisting of SEQ ID No. 2, SEQ ID No. 3 and amino acid sequences having at least 90% sequence identity with the abovementioned sequences;
with the proviso that process steps a) and b) may be carried out in either order or else in parallel.

4. Process according to Claim 3, wherein process step a) is carried out before process step b).

5. Process according to either of Claims 3 and 4, wherein polyols, polycarboxylic acids, and polyamines are formed as process products.

6. Process according to Claim 5, wherein at least one polyamine selected from the group consisting of methylene-4,4'-diamine, methylene-2,4'-diamine, methylene-2,2'-diamine, tolylene-2,4-diamine, and tolylene-2,6-diamine is formed.

7. Process according to Claim 5, wherein at least one polyol selected from the group consisting of ethylene glycol, diethylene glycol, triethylene glycol, propylene glycol, 1,2-dipropylene glycol, neopentyl glycol, glycerol, 1,1,1-trimethylolpropane, sucrose, sorbitol, and pentaerythritol is formed.

8. Process according to Claim 5, wherein at least one polycarboxylic acid selected from the group consisting of succinic acid, glutaric acid, adipic acid, phthalic acid, terephthalic acid, benzenetricarboxylic acid, oleic acid, and ricinoleic acid is formed.

9. Process according to any of Claims 3 to 8, wherein process step a) is carried out with a lipase.

10. Process according to Claim 9, wherein the lipase has an amino acid sequence as in SEQ ID No. 11 or SEQ ID No. 12 or a variant of the abovementioned sequences having at least 90% sequence identity with SEQ ID No. 11 or SEQ ID No. 12.

## Revendications

1. Utilisation d'un polypeptide ayant une séquence d'acides aminés choisie dans le groupe consistant en SEQ ID NO : 3, SEQ ID NO : 2 et les variants de ces polypeptides qui sont obtenus par addition, délétion ou substitution de jusqu'à 10 % des acides aminés contenus dans le polypeptide considéré et présentent une activité d'uréthanase, pour la dissociation enzymatique de liaisons uréthane.

2. Utilisation selon la revendication 1, le groupe uréthane étant à liaison aromatique.

3. Procédé de dégradation de polyester-polyuréthanes en des produits de dégradation à faible masse moléculaire, comprenant les étapes suivantes :
a) dissociation des groupes esters contenus dans le polyester-polyuréthane ; et
b) traitement du polyuréthane avec un polypeptide qui présente une activité d'uréthanase et a une séquence d'acides aminés choisie dans le groupe consistant en SEQ ID NO : 2, SEQ ID NO : 3 et les séquences d'acides aminés ayant une identité de séquence d'au moins 90 % avec les séquences mentionnées ci-dessus ;
à la condition que les étapes a) et b) puissent être mises en œuvre dans un ordre quelconque, ou aussi simultanément.

4. Procédé selon la revendication 3, dans lequel l'étape a) est mise en œuvre avant l'étape b).

5. Procédé selon l'une des revendications 3 ou 4, dans lequel il se forme en tant que produits du procédé des polyols, des acides polycarboxyliques et des polyamines.

6. Procédé selon la revendication 5, dans lequel au moins une polyamine est choisie dans le groupe consistant en la 4,4'-méthylènediamine, la 2,4'-méthylènediamine, la 2,2'-méthylènediamine, la 2,4-toluylènediamine et la 2,6-toluylènediamine.

7. Procédé selon la revendication 5, dans lequel au moins un polyol est choisi dans le groupe consistant en l'éthylèneglycol, le diéthylèneglycol, le triéthylèneglycol, le propylèneglycol, le 1,2-dipropylèneglycol, le néopentylglycol, le glycérol, le 1,1,1-triméthylolpropane, le saccharose, le sorbitol et le pentaérythritol.

8. Procédé selon la revendication 5, dans lequel au moins un acide polycarboxylique est choisi dans le groupe consistant en l'acide succinique, l'acide glutarique, l'acide adipique, l'acide phtalique, l'acide téréphtalique, l'acide benzènetricarboxylique, l'acide oléique et l'acide ricinoléique.

9. Procédé selon l'une des revendications 3 à 8, dans lequel l'étape a) est mise en œuvre avec une lipase.

10. Procédé selon la revendication 9, dans lequel la lipase a une séquence d'acides aminés telle que SEQ ID NO :11, SEQ ID NO : 12, ou une variante des séquences mentionnées ci-dessus ayant une identité de séquence d'au moins 90 % avec SEQ ID NO : 11 ou SEQ ID NO : 12.


### SEQUENCE LISTING

<110> Covestro Deutschland AG
<120> Neue Urethanasen für den Abbau von Polyurethanen
<130> COV 17 1 166
<160> 12
<170> PatentIn version 3.5
<210> 1
   <211> 469
   <212> PRT
   <213> Unknown
<220>
   <223> Derived from metagenome
<400> 1
   Met Met Gly Gly Val Gly Val Arg Glu Glu Leu Ala Thr Trp Thr Ala
   1               5                   10                  15
   Val Arg Leu Ala Glu His Ile Arg Lys Lys Glu Leu Ser Pro Val Glu
               20                  25                  30
   Val Thr Asp Tyr Phe Leu Arg Arg Ile Glu Ala Leu Asn Pro Ala Val
           35                  40                  45
   Asn Ala Phe Cys Thr Val Asp Ala Asp Gly Ala Met Arg Ala Ala Lys
       50                  55                  60
   Ala Ala Glu Gln Arg Leu Met Ala Gly Glu Thr Pro Pro Leu Leu Gly
   65                  70                  75                  80
   Val Pro Val Ala Ile Lys Asp Leu Thr Pro Thr Lys Gly Ile Arg Thr
                   85                  90                  95
   Thr Tyr Gly Ser Arg Leu Phe Ala Asp Asn Val Pro Glu Ala Asp Ala
               100                 105                 110
   Val Leu Val Thr Arg Leu Lys Gln Ala Gly Ala Ile Ile Val Gly Lys
           115                 120                 125
   Thr Asn Thr Pro Glu Phe Gly His Ala Gly Val Thr Asp Asn Arg Leu
       130                 135                 140
   Phe Gly Arg Thr Asn Asn Pro Trp Asp Leu Ser Arg Ile Ala Gly Gly
   145                 150                 155                 160
   Ser Ser Gly Gly Ser Asp Gly Gly Gly Ser Ile Arg Ile Pro Ala Ser
   165 170 175
   Cys Cys Gly Ile Phe Gly Phe Lys Pro Thr Phe Gly Arg Val Pro His
   180 185 190
   Asp Thr Gly Ala Thr Ala Phe Ser Ile Thr Ala Pro Phe Leu His His
   195 200 205
   Gly Pro Met Ser Arg Thr Val Glu Asp Ser Val Leu Met Leu Ala Ala
   210 215 220
   Met Gln Gly Pro Asp Gly Cys Asp Pro Phe Ser Leu Pro Leu Pro Gly
   225 230 235 240
   Ile Asp Trp Pro Leu Ser Ala Glu Ile Lys Pro Phe Ser Gln Trp Arg
   245 250 255
   Ile Ala Tyr Ser Pro Asn Leu Asp Phe Tyr Ala Ile Asp Pro Ala Val
   260 265 270
   Arg Gln Val Met Glu Gln Ala Val Ser Ala Leu Gln Gly Leu Gly Cys
   275 280 285
   Arg Val Glu Glu Val Arg Leu Gly Leu Glu Glu Gly Lys Thr Leu Val
   290 295 300
   Leu Glu Thr Phe Ala Arg Leu Trp Ala Val His Tyr Ala Ala Phe Tyr
   305 310 315 320
   Glu Glu Leu Leu Glu Arg Glu Ala Glu Leu Ser Lys Gly Phe Val Ala
   325 330 335
   Thr Ile Arg Tyr Gly Gln Gln Phe Ser Ala Val Glu Tyr Lys Arg Leu
   340 345 350
   Glu Arg Pro Arg Ala Val Val Tyr Glu Arg Val Glu Asn Val Phe Ala
   355 360 365
   Lys Tyr Asp Leu Leu Ile Thr Pro Thr Leu Ala Val Pro Pro Phe Ala
   370 375 380
   His Asp Cys Pro Pro Arg Glu Ile Asp Gly Lys Ala Val Asn Pro Tyr
   385 390 395 400
   Asn Glu Trp Met Leu Thr Ser Ile Phe Asn Leu Thr Gly His Pro Val
   405 410 415
   Ala Ser Ile Pro Ala Gly Phe Ser Pro Glu Gly Leu Pro Ile Gly Met
   420 425 430
   Gln Ile Val Gly Pro Arg Leu Ala Asp Ala Ala Val Leu Glu Phe Ala
   435 440 445
   Tyr Leu Phe Glu Gln Thr Val Ala Pro Arg Arg Pro Tyr Pro Cys Asp
   450 455 460
   Asp Val Arg Leu Asn
   465
<210> 2
   <211> 264
   <212> PRT
   <213> Unknown
<220>
   <223> Derived from metagenome
<220>
   <221> misc_feature
   <222> (252)..(252)
   <223> Xaa can be any naturally occurring amino acid
<400> 2
   Leu Asp Tyr Leu Gly Gly Phe Ser Pro Leu Glu Ser Asp Val Thr Val
   1               5                   10                  15
   Glu Lys Thr Arg Ile Ala Gly Val Pro Gly Glu Trp Ile Ser Thr Pro
               20                  25                  30
   Asp Ala Arg Lys Asp Arg Val Leu Phe Tyr Leu His Gly Gly Ala Tyr
           35                  40                  45
   Cys Phe Gly Ser Cys Asp Ser His Arg Gly Leu Val Ser Arg Leu Ala
       50                  55                  60
   Arg Ala Cys Gly Ser Arg Ala Leu Leu Ile Glu Tyr Arg Leu Ala Pro
   65                  70                  75                  80
   Glu His Pro Phe Pro Ala Ala Leu Glu Asp Ser Thr Ala Ala Tyr Arg
                   85                  90                  95
   Glu Leu Ile Arg Ser Gly Val Arg Pro Glu Asn Leu Val Ile Ala Gly
               100                 105                 110
   Asp Ser Ala Gly Gly Gly Leu Thr Met Ala Thr Leu Leu Thr Leu Arg
           115                 120                 125
   Asp Glu Gly Asp Pro Leu Pro Ser Ala Ala Val Leu Leu Ser Pro Trp
   130 135 140
   Thr Asp Leu Glu Gly Thr Gly Glu Ser Met Lys Thr Lys Ala Asp Val
   145 150 155 160
   Glu Pro Trp Leu Asp Pro Glu Lys Ser His Leu Leu Ala Lys Leu Tyr
   165 170 175
   Leu Gly Asp Leu Asp Pro Arg His Pro Leu Val Ser Pro Ile His Ala
   180 185 190
   Asp Leu Asn Asn Leu Pro Pro Leu Leu Val His Val Gly Ser Asp Glu
   195 200 205
   Cys Leu Leu Asp Asp Ser Val Arg Leu Val Glu Arg Ala Lys Ser Ala
   210 215 220
   Gly Val Glu Thr Glu Phe Lys Ile Cys Asp Glu Met Trp His Val Phe
   225 230 235 240
   His Gly Phe Pro Ile Pro Glu Ala Gln Gln Ala Xaa Glu Glu Ile Gly
   245 250 255
   Ala Phe Val Arg Ala Arg Leu Pro
   260
<210> 3
   <211> 297
   <212> PRT
   <213> Unknown
<220>
   <223> Derived from metagenome
<400> 3
   Met Ala Ser Pro Gln Ser Glu Ala Ile Arg Gln Met Leu Arg Glu Gln
   1 5 10 15
   Lys Glu Ala Ala Lys Lys Gly Ala Pro Ser Ile Glu Glu Gln Arg Arg
   20 25 30
   Gln Leu Asp Tyr Leu Gly Gly Phe Ser Pro Leu Glu Ser Asp Val Thr
   35 40 45
   Val Glu Lys Thr Arg Ile Ala Gly Val Pro Gly Glu Trp Ile Ser Thr
   50 55 60
   Pro Asp Ala Arg Lys Asp Arg Val Leu Phe Tyr Leu His Gly Gly Ala
   65                  70                  75                  80
   Tyr Cys Phe Gly Ser Cys Asp Ser His Arg Gly Leu Val Ser Arg Leu
                   85                  90                  95
   Ala Arg Ala Cys Gly Ser Arg Ala Leu Leu Ile Glu Tyr Arg Leu Ala
               100                 105                 110
   Pro Glu His Pro Phe Pro Ala Ala Leu Glu Asp Ser Thr Ala Ala Tyr
           115                 120                 125
   Arg Glu Leu Ile Arg Ser Gly Val Arg Pro Glu Asn Leu Val Ile Ala
       130                 135                 140
   Gly Asp Ser Ala Gly Gly Gly Leu Thr Met Ala Thr Leu Leu Thr Leu
   145                 150                 155                 160
   Arg Asp Glu Gly Asp Pro Leu Pro Ser Ala Ala Val Leu Leu Ser Pro
                   165                 170                 175
   Trp Thr Asp Leu Glu Gly Thr Gly Glu Ser Met Lys Thr Lys Ala Asp
               180                 185                 190
   Val Glu Pro Trp Leu Asp Pro Glu Lys Ser His Leu Leu Ala Lys Leu
           195                 200                 205
   Tyr Leu Gly Asp Leu Asp Pro Arg His Pro Leu Val Ser Pro Ile His
       210                 215                 220
   Ala Asp Leu Asn Asn Leu Pro Pro Leu Leu Val His Val Gly Ser Asp
   225                 230                 235                 240
   Glu Cys Leu Leu Asp Asp Ser Val Arg Leu Val Glu Arg Ala Lys Ser
                   245                 250                 255
   Ala Gly Val Glu Thr Glu Phe Lys Ile Trp Asp Glu Met Trp His Val
               260                 265                 270
   Phe His Gly Phe Pro Ile Pro Glu Ala Gln Gln Ala Ile Glu Glu Ile
           275                 280                 285
   Gly Ala Phe Val Arg Ala Arg Leu Pro
       290                 295
<210> 4
   <211> 311
   <212> PRT
   <213> Unknown
<220>
   <223> Derived from metagenome
<400> 4
   Met Ala Asp Pro Gln Leu Glu Ala Val Leu Val Gly Leu Ala Gln Ala
   1               5                   10                  15
   Ser Ala Gly Ala Gln Gly Pro Ala Thr Val Glu Gly Phe Arg Val Ala
               20                  25                  30
   Leu Arg Glu Leu Thr Arg Met Leu Asp Phe Arg Asp Ile Pro Val Gly
           35                  40                  45
   Arg Val Glu Asn Arg Met Ile Pro Gly Pro Asp Gly Glu Ile Gly Ile
       50                  55                  60
   Arg Ile Tyr Thr Pro Ile Ala Ala Gly Ala Arg Met Leu Glu Thr Leu
   65                  70                  75                  80
   Ile Tyr Phe His Gly Gly Gly Phe Val Ala Gly Asp Leu Glu Thr His
                   85                  90                  95
   Asp Thr Leu Cys Arg Gly Leu Thr Ala Arg Ser Gly Cys Arg Val Ile
               100                 105                 110
   Ser Val Asp Tyr Arg Leu Ala Pro Glu His Pro Phe Pro Ala Ala Ile
           115                 120                 125
   Asp Asp Ser Tyr Ala Ala Leu Arg Trp Ile Glu Ala Asn Ala Thr Thr
       130                 135                 140
   Leu Gly Val Asp Ser Asn Arg Ile Ala Val Gly Gly Asp Ser Ala Gly
   145                 150                 155                 160
   Gly Asn Ile Ala Ala Val Val Ala Gln Leu Ala Arg Gly Ala Gly Asn
                   165                 170                 175
   Pro Val Val Arg Phe Gln Leu Leu Ile Tyr Pro Val Val Gln Trp Asp
               180                 185                 190
   Val Ala Thr Pro Ser Arg Gln Gln Phe Ala Glu Asp Pro Ile Ile Pro
           195                 200                 205
   Arg Asp Val Ile Asp Met Cys Ala Arg Asn Tyr Phe Gly Pro Met Val
       210                 215                 220
   Pro Ala Thr Asp Phe Arg Ala Ala Pro Leu Ala Ala Ser Asp Leu Ala
   225                 230                 235                 240
   Gly Leu Pro Pro Ala Tyr Val Ile Thr Ala Gly Leu Asp Pro Leu Arg
                   245                 250                 255
   Asp Glu Gly Ala Gln Tyr Ala Glu Lys Leu Arg Glu Ala Gly Val Ala
               260                 265                 270
   Val Glu His Val Gly Tyr Asp Asp Met Ile His Gly Phe Met Ser Met
           275                 280                 285
   Ser Asn Ala Leu Asp Thr Ala Lys Leu Ala Ile Glu Arg Ala Gly Asp
       290                 295                 300
   Ala Leu Arg Asn Ala Leu Arg
   305                 310
<210> 5
   <211> 316
   <212> PRT
   <213> Unknown
<220>
   <223> Derived from metagenome
<400> 5
   Met Ser Leu Asp Pro Lys Ala Arg Glu Leu Leu Ala Met Val Tyr Arg
   1               5                   10                  15
   Val Asn Ala Pro Arg Phe His Glu Leu Ser Val Ser Gln Ala Arg His
               20                  25                  30
   Ala Thr Gln Lys Leu Met Phe Ala Phe Arg Pro Glu Ala Pro Ala Val
           35                  40                  45
   Ala Ser Thr Thr Glu Val Pro Ile Pro Arg Pro Asp Gly Ser Val Leu
       50                  55                  60
   Phe Ala Arg Leu Tyr Arg Pro Leu Gly Cys His Ala Ser Glu Asp Leu
   65                  70                  75                  80
   Gly Leu Leu Ile Tyr Phe His Gly Gly Gly Trp Cys Thr Gly Asp Leu
                   85                  90                  95
   Pro Gly Tyr Asp Val Leu Cys Arg Glu Leu Ala Asn Gln Ser Gly Ala
               100                 105                 110
   Ala Val Leu Ser Val Asp Tyr Arg Leu Ala Pro Glu His Arg Phe Pro
   115 120 125
   Ala Ala Val His Asp Ala Ser Leu Ala Phe Glu Trp Ser Thr Glu Asn
   130 135 140
   Ala Ser Leu Leu Gly Val Asp Ala Glu Arg Ile Ala Leu Gly Gly Asp
   145 150 155 160
   Ser Ala Gly Gly Asn Leu Ala Ile Val Ala Ala Leu Glu Ala Arg Asp
   165 170 175
   Arg Ala Ala Arg Met Pro Arg Ala Leu Ala Leu Ile Tyr Pro Ser Thr
   180 185 190
   Gln Ile His Ser Glu Arg Ser Ser Arg Glu Thr Phe Ala Asp Gly Tyr
   195 200 205
   Phe Leu Asp Arg Glu Ser Leu Arg Trp Phe Tyr Glu His Tyr Phe Ala
   210 215 220
   Asp Pro Ala Gln Ala Gln Ser Trp Gln Ala Ser Pro Met Leu Ala Ala
   225 230 235 240
   Ser Leu Ala Gly Leu Pro Pro Ala Ile Leu Ile Thr Ala Gly Cys Asp
   245 250 255
   Pro Leu Thr Asp Asp Cys Val Ala Phe Ala Glu Arg Met Val Ala Asp
   260 265 270
   Gly Gly Leu Val Val Arg His His Phe Glu Gly Met Val His Gly Phe
   275 280 285
   Leu Pro Leu Gly Lys Phe Phe Ala Gln Ala Asn Glu Ala Val Arg Cys
   290 295 300
   Val Ser Ser Tyr Leu Arg Glu Ala Leu Gln Ala Ser
   305 310 315
<210> 6
   <211> 297
   <212> PRT
   <213> Unknown
<220>
   <223> Derived from metagenome
<220>
   <221> misc_feature
   <222> (216)..(216)
   <223> Xaa can be any naturally occurring amino acid
<400> 6
   Met Ser Leu Glu Glu Leu Ala Val Val Arg Gln Leu Leu Ala Gly Leu
   1               5                   10                  15
   Val Thr Gly Glu Ala Arg Ser Leu Glu Asp Phe Arg Thr Ser Tyr Asp
               20                  25                  30
   Glu Ala Gly Lys Ala Phe Gly Leu Pro Glu Gly Val Thr Val Thr Pro
           35                  40                  45
   Val Ser Ala Gly Gly Val Pro Gly Glu Trp Leu Ala Pro Ala Ala Gly
       50                  55                  60
   Ala Gly Lys Arg Val Leu Leu Tyr Leu His Gly Gly Gly Tyr Ala Leu
   65                  70                  75                  80
   Gly Ser Leu Asp Ser His Arg His Leu Ala Ala His Thr Ala Leu Ala
                   85                  90                  95
   Leu Asn Gly Arg Val Leu Leu Ile Asp Tyr Arg Arg Ser Pro Glu His
               100                 105                 110
   Pro Phe Pro Ala Ala Val Asp Asp Ala Leu Ala Ala Tyr Arg Trp Leu
           115                 120                 125
   Thr Glu Thr Gly Val Asp Pro Ala Lys Leu Ala Val Ala Gly Asp Ser
       130                 135                 140
   Ala Gly Gly Gly Leu Thr Val Ala Val Leu Leu Ala Ala Arg Asp Ala
   145                 150                 155                 160
   Gly Leu Arg Leu Pro Ala Ala Ala Val Cys Ile Ser Pro Trp Ala Asn
                   165                 170                 175
   Leu Glu Asn Lys Gly Ala Ser Tyr Gly Ala Lys Ala Asn Val Asp Pro
               180                 185                 190
   Met Val Arg His Ala Asp Leu Glu Leu Trp Thr Ala Ala Tyr Leu Gly
           195                 200                 205
   Thr Ser Thr Pro Arg Arg Ala Xaa Leu Ala Ser Pro Val Tyr Ala Asp
       210                 215                 220
   Leu Asn Gly Leu Pro Pro Phe Leu Ile Gln Val Gly Ser Ser Glu Val
   225                 230                 235                 240
   Leu Leu Ser Asp Ser His Leu Leu Ala Asp Arg Leu Lys Glu Ala Gly
   245 250 255
   Val Ser Val Asp Leu His Val Trp Pro Glu Met Ile His Val Trp His
   260 265 270
   Trp Phe Ala Pro Val Leu Ser Glu Gly Arg Ala Ala Ile Asp Glu Met
   275 280 285
   Ala Ser Phe Leu Asp Thr Lys Leu Gly
   290 295
<210> 7
   <211> 490
   <212> PRT
   <213> Unknown
<220>
   <223> Derived from metagenome
<400> 7
   Met Thr Gly Leu His Phe Arg Ser Ala Ser Glu Leu Gly Arg Met Ile
   1 5 10 15
   Arg Arg Gly Glu Ile Ser Ser Ala Glu Leu Thr Asp His Phe Ile Gln
   20 25 30
   Arg Ile Glu Thr Leu Asp Gly Lys Thr Asn Ala Val Val Ala Arg Asp
   35 40 45
   Phe Asp Arg Ala Arg Ala Leu Ala Lys Glu Ala Asp Ala Ala Gln Ala
   50 55 60
   Arg Gly Ala Ser Leu Gly Ala Leu His Gly Leu Pro Phe Thr Ile Lys
   65 70 75 80
   Asp Ala Tyr Glu Val Glu Gly Ile Val Ser Thr Gly Gly Asn Pro Thr
   85 90 95
   Trp Lys Asp His Val Pro Thr Ser Ser Ala Thr Ala Val Glu Arg Leu
   100 105 110
   Gln Arg Ser Gly Ala Ile Val Met Gly Lys Thr Asn Val Pro Tyr Leu
   115 120 125
   Ser Gly Asp Leu Gln Thr Tyr Asn Asp Ile Tyr Gly Thr Thr Asn Asn
   130 135 140
   Pro Trp Ala Leu Asp Cys Gly Pro Gly Gly Ser Ser Gly Gly Ser Ala
   145                 150                 155                 160
   Ala Ser Leu Ala Ala Gly Phe Ala Ala Ala Glu Phe Gly Ser Asp Ile
                   165                 170                 175
   Gly Gly Ser Ile Arg Thr Pro Ala His Leu Cys Gly Val Phe Gly His
               180                 185                 190
   Lys Pro Ser Phe Gly Ile Val Pro Lys Arg Gly His Leu Ser Pro Pro
           195                 200                 205
   Pro Gly Cys Leu Ser Glu Gly Asp Leu Ser Val Ala Gly Pro Leu Ala
       210                 215                 220
   Arg Ser Ala Glu Asp Leu Lys Leu Leu Leu Ser Leu Thr Ala Gly Pro
   225                 230                 235                 240
   Asp Trp Ala Asp Ala Ala Gly Trp Lys Leu Asp Leu Pro Pro Ala Arg
                   245                 250                 255
   Ala Arg Thr Pro Arg Glu Leu Arg Ala Ala Val Trp Ile Asp Asp Glu
               260                 265                 270
   Phe Cys Asp Ile Asp Arg Glu Ser Ala Asp Leu Leu Arg Asn Ala Ala
           275                 280                 285
   Lys Ala Leu Gln Asp Ala Gly Ala Asn Val Asp Trp Asn Ala Arg Pro
       290                 295                 300
   Asp Phe Thr Leu Ala Glu Ile Thr Glu Cys Tyr Leu Ile Leu Leu His
   305                 310                 315                 320
   Ser Gln Ile Gly Ala Gly Met Pro Gln Ser Ile Arg Asp His Trp Ala
                   325                 330                 335
   Glu Met Lys Lys Gly Phe Ala Pro Asp Asp Lys Ser His Ala Ala Leu
               340                 345                 350
   Gln Ala Ile Gly Gly Thr Leu Ser Leu Ala Glu Arg Ala Val Trp Lys
           355                 360                 365
   Glu Val Gln Ala Gln Leu Arg Trp Lys Trp His Thr Phe Phe Lys Ser
       370                 375                 380
   Tyr Asp Val Val Leu Ser Pro Val Leu Met Arg Pro Ala Phe Glu His
   385                 390                 395                 400
   Asn His Gln Ser Asn Trp His Lys Arg Glu Leu Asp Val Asn Gly Val
   405 410 415
   Lys Arg Pro Tyr Met Asp Val Leu Ile Trp Ala Gly Pro Ala Val Val
   420 425 430
   Ser Tyr Leu Pro Ala Thr Ala Ala Pro Val Gly Val Thr Ser Glu Gly
   435 440 445
   Lys Pro Val Gly Ile Gln Ile Ile Gly Pro His Leu Glu Asp Tyr Thr
   450 455 460
   Thr Ile Ala Val Ala Gly Met Phe Glu Glu Ile Leu Gly Gly Phe Lys
   465 470 475 480
   Pro Pro Lys Gly Trp Ala Ala Ala Leu Glu
   485 490
<210> 8
   <211> 177
   <212> PRT
   <213> Unknown
<220>
   <223> Derived from metagenome
<400> 8
   Cys Ala Cys Cys Leu Ser Leu Val Asp Arg Asp Gly Arg Arg Pro Gly
   1 5 10 15
   Glu Leu Ala Val Ala Gly Asp Ser Ala Gly Gly Gly Leu Thr Val Ala
   20 25 30
   Val Leu Leu Ala Ala Arg Asp Ala Gly Leu Arg Leu Pro Ala Ala Ala
   35 40 45
   Val Cys Ile Ser Pro Trp Ala Asn Leu Glu Asn Lys Gly Ala Ser Tyr
   50 55 60
   Gly Ala Lys Ala Asn Val Asp Pro Met Val Arg His Ala Asp Leu Glu
   65 70 75 80
   Leu Trp Thr Ala Ala Tyr Leu Gly Thr Ser Thr Pro Arg Arg Ala Pro
   85 90 95
   Leu Ala Ser Pro Val Tyr Ala Asp Leu Asn Gly Leu Pro Pro Phe Leu
   100 105 110
   Ile Gln Val Gly Ser Ser Glu Val Leu Leu Ser Asp Ser His Leu Leu
           115                 120                 125
   Ala Asp Arg Leu Lys Glu Ala Gly Val Ser Val Asp Leu His Val Trp
       130                 135                 140
   Pro Glu Met Ile His Val Trp His Trp Phe Ala Pro Val Leu Ser Glu
   145                 150                 155                 160
   Gly Arg Ala Ala Ile Asp Glu Met Ala Ser Phe Leu Asp Thr Lys Leu
                   165                 170                 175
   Gly
<210> 9
   <211> 491
   <212> PRT
   <213> Unknown
<220>
   <223> Derived from metagenome
<400> 9
   Leu Glu Arg Ser Asp Leu Asp Tyr Ala Ser Ala Thr Glu Ile Ala Arg
   1               5                   10                  15
   Leu Val Arg Thr Arg Gln Ile Ser Ala Ala Asp Val Thr Glu His Ala
               20                  25                  30
   Ile Ser Arg Ile Glu Ala Arg Asn Gly Ser Leu Asn Ala Phe Val Tyr
           35                  40                  45
   Thr Asp Phe Glu Gln Ala Arg Ser Arg Ala Lys Asp Leu Asp Thr Arg
       50                  55                  60
   Ile Ser Ala Gly Glu Asp Val Gly Pro Leu Ala Gly Val Pro Thr Ala
   65                  70                  75                  80
   Ile Lys Asp Leu Phe Asn Phe Tyr Pro Gly Trp Pro Ser Thr Leu Gly
                   85                  90                  95
   Gly Ile Arg Cys Leu Arg Asp Phe Lys Leu Asp Val Lys Ser Arg Tyr
               100                 105                 110
   Ala Thr Lys Met Glu Glu Ala Gly Ala Val Val Leu Gly Ile Thr Asn
           115                 120                 125
   Ser Pro Val Leu Gly Phe Arg Gly Thr Thr Asp Asn Asp Leu Tyr Gly
   130 135 140
   Pro Thr Arg Asn Pro Phe Asp Leu Ser Arg Asn Ser Gly Gly Ser Ser
   145 150 155 160
   Gly Gly Thr Ser Ala Ala Val Ala Asp Gly Leu Leu Pro Ile Gly Asp
   165 170 175
   Gly Thr Asp Gly Gly Gly Ser Ile Arg Ile Pro Ala Ala Trp Cys His
   180 185 190
   Val Phe Gly Phe Gln Ala Ser Pro Gly Arg Ile Pro Leu Ala Ile Arg
   195 200 205
   Pro Asn Ala Phe Gly Ala Ala Ala Pro Phe Ile Tyr Glu Gly Pro Ile
   210 215 220
   Thr Arg Thr Val Glu Asp Ala Ala Leu Ala Met Ser Val Leu Ala Gly
   225 230 235 240
   Ser Asp Pro Ala Asp Pro Phe Ser Leu Asn Asp Arg Leu Asp Trp Leu
   245 250 255
   Gly Ala Val Asp Gln Pro Ile Thr Ser Leu Arg Ile Gly Phe Thr Pro
   260 265 270
   Asp Phe Gly Gly Phe Pro Val Glu Pro Ala Val Ala Ala Thr Ile Ala
   275 280 285
   His Ala Val Arg Ala Phe Glu Gln Ala Gly Ala Lys Ile Val Pro Leu
   290 295 300
   Lys Leu Asp Phe Gly Tyr Thr His Asp Glu Leu Ser Gln Leu Trp Cys
   305 310 315 320
   Arg Met Ile Ser Gln Gly Thr Ile Ala Val Val Asp Ser Phe Ala Glu
   325 330 335
   Asn Gly Leu His Leu Glu Pro Asp Phe Pro Ala Pro Val Met Glu Trp
   340 345 350
   Ala Gln Lys Ala Lys Asn Ala Thr Pro Leu Asp Leu His Arg Asp Gln
   355 360 365
   Val Met Arg Thr Lys Val Tyr Asp Val Leu Asn Ala Ala Phe Ser Gln
   370 375 380
   Val Asp Leu Ile Ala Gly Pro Thr Thr Thr Cys Leu Pro Thr Pro Asn
   385 390 395 400
   Gly Glu Arg Gly Met Thr Val Gly Pro Ser Glu Ile Ala Gly Thr Pro
   405 410 415
   Ile Asn Arg Leu Ile Gly Phe Cys Pro Thr Phe Leu Thr Asn Phe Thr
   420 425 430
   Gly Asn Pro Ala Ala Ser Leu Pro Ala Gly Leu Ala Asp Gly Leu Pro
   435 440 445
   Val Gly Leu Met Leu Ile Gly Pro Arg Arg Asp Asp Leu Thr Val Leu
   450 455 460
   Ser Ala Ser Ala Ala Phe Glu Arg Val Gln Pro Trp Ala Asp Ser Tyr
   465 470 475 480
   Arg Ile Pro Ala Ala Arg Pro Leu Gly Ser Gln
   485 490
<210> 10
   <211> 330
   <212> PRT
   <213> Unknown
<220>
   <223> Derived from metagenome
<400> 10
   Met Arg Pro Arg Ser Arg Pro His Ala Arg Ala Arg Gly Ala Pro Thr
   1               5                   10                  15
   Ile Leu Arg Asp Pro Ala Thr Met Ala Leu His Arg Thr Pro Arg Arg
               20                  25                  30
   Asn Asp Met Ala Asp Arg Gly Ile Glu Val Val His Ala His Leu Ala
           35                  40                  45
   Lys Leu Pro Pro Ala Asp Ser Leu Thr Val Ala Glu Arg Arg Ala Gln
       50                  55                  60
   Tyr Glu Arg Ala Glu Lys Val Phe Pro Leu Ser Pro Asp Val Lys Val
   65                  70                  75                  80
   Glu Arg Val Thr Ala Gly Ala Ala Pro Ala Glu Trp Leu Arg Pro Pro
                   85                  90                  95
   Ser Ala Arg Ala Gly His Val Val Leu Tyr Leu His Gly Gly Gly Tyr
                   100                 105                 110
   Val Ile Gly Ser Pro Arg Ser His Arg His Leu Ala Ala Ala Ile Ala
           115                 120                 125
   Gly Ala Ala Gly Thr Asn Ala Leu Leu Leu Asp Tyr Arg Leu Ala Pro
       130                 135                 140
   Glu His Pro Phe Pro Ala Ala Leu Asp Asp Ala Val Ala Ala Tyr Arg
   145                 150                 155                 160
   Trp Leu Leu Asp Gln Gly Ile Ala Ala Glu His Ile Ala Val Ala Gly
                   165                 170                 175
   Asp Ser Ala Gly Gly Gly Leu Thr Val Ala Thr Leu Leu Ala Leu Arg
               180                 185                 190
   Asp Ala His Leu Pro Arg Pro Ala Ala Gly Val Cys Ile Ser Pro Trp
           195                 200                 205
   Val Asp Leu Thr Cys Ser Gly Gly Ser Tyr Gln Ser Lys Ala Gly Val
       210                 215                 220
   Asp Pro Ile Val Arg Gln Ala Gly Val Ala Glu Met Ala Arg Ala Tyr
   225                 230                 235                 240
   Leu Gly Ala Thr Asp Pro Arg Ser Pro Leu Ala Ser Pro Leu Phe Ala
                   245                 250                 255
   Asp Leu Arg Gly Leu Pro Pro Leu Leu Ile His Val Gly Ser Asp Glu
               260                 265                 270
   Val Leu Leu Asp Asp Ala Ile Gly Leu Ala Glu Arg Ala Lys Ala Ala
           275                 280                 285
   Gly Val Asp Ala Thr Leu Glu Gln Trp Asp Arg Met Ile His Val Trp
       290                 295                 300
   His Trp Phe Leu Pro Met Leu Asp Glu Ala Gln Thr Ala Val Glu Ser
   305                 310                 315                 320
   Ile Gly Arg Phe Val Arg Ala Arg Thr Ala
                   325                 330
<210> 11
   <211> 544
   <212> **PRT**
   <213> Pig
<400> 11
   Gly Gln Pro Ala Ser Pro Pro Val Val Asp Thr Ala Gln Gly Arg Val
   1               5                   10                  15
   Leu Gly Lys Tyr Val Ser Leu Glu Gly Leu Ala Gln Pro Val Ala Val
               20                  25                  30
   Phe Leu Gly Val Pro Phe Ala Lys Pro Pro Leu Gly Ser Leu Arg Phe
           35                  40                  45
   Ala Pro Pro Gln Pro Ala Glu Pro Trp Ser Phe Val Lys Asn Thr Thr
       50                  55                  60
   Ser Tyr Pro Pro Met Cys Cys Gln Asp Pro Val Ala Gly Gln Met Thr
   65                  70                  75                  80
   Ser Asp Leu Phe Thr Asn Arg Lys Glu Arg Leu Ile Pro Glu Phe Ser
                   85                  90                  95
   Glu Asp Cys Leu Tyr Leu Asn Ile Tyr Thr Pro Ala Asp Leu Thr Lys
               100                 105                 110
   Arg Gly Arg Leu Pro Val Met Val Trp Ile His Gly Gly Gly Leu Val
           115                 120                 125
   Val Gly Gly Ala Ser Thr Tyr Asp Gly Leu Ala Leu Ala Ala His Glu
       130                 135                 140
   Asn Val Val Val Val Ala Ile Gln Tyr Arg Leu Gly Ile Trp Gly Phe
   145                 150                 155                 160
   Phe Ser Thr Gly Asp Glu His Ser Arg Gly Asn Trp Gly His Leu Asp
                   165                 170                 175
   Gln Val Ala Ala Leu His Trp Val Gln Glu Asn Ile Ala Asn Phe Gly
               180                 185                 190
   Gly Asp Pro Gly Ser Val Thr Ile Phe Gly Glu Ser Ala Gly Gly Glu
           195                 200                 205
   Ser Val Ser Val Leu Val Leu Ser Pro Leu Ala Lys Asn Leu Phe His
       210                 215                 220
   Arg Ala Ile Ser Glu Ser Gly Val Ala Phe Thr Ala Gly Leu Val Arg
   225                 230                 235                 240
   Lys Asp Met Lys Ala Ala Ala Lys Gln Ile Ala Val Leu Ala Gly Cys
                   245                 250                 255
   Lys Thr Thr Thr Ser Ala Val Phe Val His Cys Leu Arg Gln Lys Ser
               260                 265                 270
   Glu Asp Glu Leu Leu Asp Leu Thr Leu Lys Met Lys Phe Phe Ala Leu
           275                 280                 285
   Asp Leu His Gly Asp Pro Arg Glu Ser His Pro Phe Leu Thr Thr Val
       290                 295                 300
   Val Asp Gly Val Leu Leu Pro Lys Met Pro Glu Glu Ile Leu Ala Glu
   305                 310                 315                 320
   Lys Asp Phe Asn Thr Val Pro Tyr Ile Val Gly Ile Asn Lys Gln Glu
                   325                 330                 335
   Phe Gly Trp Leu Leu Pro Thr Met Met Gly Phe Pro Leu Ser Glu Gly
               340                 345                 350
   Lys Leu Asp Gln Lys Thr Ala Thr Ser Leu Leu Trp Lys Ser Tyr Pro
           355                 360                 365
   Ile Ala Asn Ile Pro Glu Glu Leu Thr Pro Val Ala Thr Asp Lys Tyr
       370                 375                 380
   Leu Gly Gly Thr Asp Asp Pro Val Lys Lys Lys Asp Leu Phe Leu Asp
   385                 390                 395                 400
   Leu Met Gly Asp Val Val Phe Gly Val Pro Ser Val Thr Val Ala Arg
                   405                 410                 415
   Gln His Arg Asp Ala Gly Ala Pro Thr Tyr Met Tyr Glu Phe Gln Tyr
               420                 425                 430
   Arg Pro Ser Phe Ser Ser Asp Lys Lys Pro Lys Thr Val Ile Gly Asp
           435                 440                 445
   His Gly Asp Glu Ile Phe Ser Val Phe Gly Ala Pro Phe Leu Arg Gly
       450                 455                 460
   Asp Ala Pro Glu Glu Glu Val Ser Leu Ser Lys Thr Val Met Lys Phe
   465                 470                 475                 480
   Trp Ala Asn Phe Ala Arg Ser Gly Asn Pro Asn Gly Glu Gly Leu Pro
                   485                 490                 495
   His Trp Pro Met Tyr Asp Gln Glu Glu Gly Tyr Leu Gln Ile Gly Val
   500 505 510
   Asn Thr Gln Ala Ala Lys Arg Leu Lys Gly Glu Glu Val Ala Phe Trp
   515 520 525
   Asn Asp Leu Leu Ser Lys Glu Ala Ala Lys Lys Pro Pro Lys Ile Lys
   530 535 540
<210> 12
   <211> 342
   <212> PRT
   <213> Candida sp.
<400> 12
   Met Lys Leu Leu Ser Leu Thr Gly Val Ala Gly Val Leu Ala Thr Cys
   1 5 10 15
   Val Ala Ala Thr Pro Leu Val Lys Arg Leu Pro Ser Gly Ser Asp Pro
   20 25 30
   Ala Phe Ser Gln Pro Lys Ser Val Leu Asp Ala Gly Leu Thr Cys Gln
   35 40 45
   Gly Ala Ser Pro Ser Ser Val Ser Lys Pro Ile Leu Leu Val Pro Gly
   50 55 60
   Thr Gly Thr Thr Gly Pro Gln Ser Phe Asp Ser Asn Trp Ile Pro Leu
   65 70 75 80
   Ser Thr Gln Leu Gly Tyr Thr Pro Cys Trp Ile Ser Pro Pro Pro Phe
   85 90 95
   Met Leu Asn Asp Thr Gln Val Asn Thr Glu Tyr Met Val Asn Ala Ile
   100 105 110
   Thr Ala Leu Tyr Ala Gly Ser Gly Asn Asn Lys Leu Pro Val Leu Thr
   115 120 125
   Trp Ser Gln Gly Gly Leu Val Ala Gln Trp Gly Leu Thr Phe Phe Pro
   130 135 140
   Ser Ile Arg Ser Lys Val Asp Arg Leu Met Ala Phe Ala Pro Asp Tyr
   145 150 155 160
   Lys Gly Thr Val Leu Ala Gly Pro Leu Asp Ala Leu Ala Val Ser Ala
   165 170 175
   Pro Ser Val Trp Gln Gln Thr Thr Gly Ser Ala Leu Thr Thr Ala Leu
               180                 185                 190
   Arg Asn Ala Gly Gly Leu Thr Gln Ile Val Pro Thr Thr Asn Leu Tyr
           195                 200                 205
   Ser Ala Thr Asp Glu Ile Val Gln Pro Gln Val Ser Asn Ser Pro Leu
       210                 215                 220
   Asp Ser Ser Tyr Leu Phe Asn Gly Lys Asn Val Gln Ala Gln Ala Val
   225                 230                 235                 240
   Cys Gly Pro Leu Phe Val Ile Asp His Ala Gly Ser Leu Thr Ser Gln
                   245                 250                 255
   Phe Ser Tyr Val Val Gly Arg Ser Ala Leu Arg Ser Thr Thr Gly Gln
               260                 265                 270
   Ala Arg Ser Ala Asp Tyr Gly Ile Thr Asp Cys Asn Pro Leu Pro Ala
           275                 280                 285
   Asn Asp Leu Thr Pro Glu Gln Lys Val Ala Ala Ala Ala Leu Leu Ala
       290                 295                 300
   Pro Ala Ala Ala Ala Ile Val Ala Gly Pro Lys Gln Asn Cys Glu Pro
   305                 310                 315                 320
   Asp Leu Met Pro Tyr Ala Arg Pro Phe Ala Val Gly Lys Arg Thr Cys
                   325                 330                 335
   Ser Gly Ile Val Thr Pro
               340